# FASCICULE DE BREVET EUROPEEN

(11) **EP 4 448 065 B1**
(45) Date de publication et mention de la délivrance du brevet: **04.02.2026**
(21) Numéro de dépôt: 22844250.5
(22) Date de dépôt: 09.12.2022
(51) Int. Cl.: A61M 11/00, A61M 15/08, B05B 11/10

(54) **DISPOSITIF DE DISTRIBUTION DE PRODUIT FLUIDE ET SON PROCEDE D'AMORÇAGE**
VORRICHTUNG ZUR ABGABE EINES FLÜSSIGEN PRODUKTS UND VERFAHREN ZUM FÜLLEN DAVON
DEVICE FOR DISPENSING A FLUID PRODUCT AND METHOD FOR PRIMING SAME

(30) Priorité: 13.12.2021 FR 2113413
(43) Date de publication de la demande: 23.10.2024
(73) Titulaire: APTAR France SAS, 27110 Le Neubourg (FR)
(72) Inventeur: SAVALLE, Matthieu, 76350 OISSEL (FR)
(74) Mandataire: CAPRI
(86) Numéro de dépôt international: PCT/FR2022/052301
(87) Numéro de publication internationale: WO 2023/111429

(56) Documents cités:
- US-A- 5 893 484
- US-A1- 2007 095 853
- US-A1- 2009 236 445

## Description

La présente invention concerne un dispositif de distribution de produit fluide, notamment pour une distribution nasale d'une ou deux doses de produit fluide pharmaceutique. La présente invention concerne également un procédé d'amorçage d'un dispositif de distribution de produit fluide.

Un inconvénient de certains dispositifs de distribution nasale de produit fluide, en particulier ceux utilisant une pompe avec une chambre de dosage pour définir le volume de la dose distribuée à chaque actionnement, concerne l'obligation d'amorcer la pompe pour remplacer l'air contenu initialement dans la chambre de dosage par du fluide. Cet amorçage est généralement réalisé par un ou plusieurs actionnements du dispositif, avec par conséquent une perte possible de produit fluide.

La présente invention a pour but de fournir un dispositif de distribution de produit fluide et son procédé d'amorçage qui ne reproduisent pas les inconvénients susmentionnés.

Plus particulièrement, la présente invention a pour but de fournir un tel dispositif et procédé qui ne nécessitent aucun amorçage de la pompe.

La présente invention a également pour but de fournir un tel dispositif et procédé qui soient simples et peu coûteux à fabriquer et à assembler. US 2009/236445 A1 concerne un distributeur de fluide, par exemple pour un spray nasal, et concerne particulièrement, mais pas exclusivement, un distributeur de fluide pour l'administration de médicaments.

La présente invention a donc pour objet un dispositif de produit fluide comprenant un distributeur assemblé sur un réservoir contenant du produit fluide, ledit distributeur comportant un corps de pompe contenant une chambre de dosage, et une tête de distribution comportant un orifice de distribution, ladite tête de distribution étant déplaçable axialement par rapport audit corps de pompe entre une position de repos et une position d'actionnement, un piston, solidaire de ladite tête de distribution, étant adapté à coulisser dans ladite chambre de dosage entre une position de repos et une position d'actionnement, ladite chambre de dosage comportant au moins deux ouvertures, une première ouverture permettant le remplissage de ladite chambre de dosage et une seconde ouverture reliant ladite chambre de dosage audit orifice de distribution, ladite première ouverture étant réalisée dans une partie cylindrique dudit corps de pompe dans laquelle ledit piston coulisse lors de l'actionnement, ladite première ouverture reliant ladite chambre de dosage audit réservoir lorsque ledit piston est en position de repos, et ladite première ouverture isolant ladite chambre de dosage dudit réservoir lorsque ledit piston se déplace hors de sa position de repos, ladite chambre de dosage étant remplie à travers ladite première ouverture lorsque ledit distributeur est assemblé sur ledit réservoir. L'invention est définie par le jeu de revendications.

Avantageusement, un bord inférieur de ladite première ouverture est disposé au repos à une distance d1 dudit piston, de sorte que, lors de l'actionnement, ladite première ouverture est fermée après une course initiale correspondant à ladite distance d1.

Avantageusement, ladite distance d1 forme moins de 30% de la course d'actionnement totale dudit piston, de préférence environ 20%.

Avantageusement, ladite première ouverture comporte une ou plusieurs fentes formée(s) dans la partie cylindrique dudit corps de pompe.

Avantageusement, un insert creux est disposé dans ladite tête de distribution, ledit insert creux définissant un canal d'expulsion reliant lors de l'actionnement ladite chambre de dosage audit orifice de distribution, ledit insert creux comportant ledit piston.

Avantageusement, ledit piston est formé par un joint, notamment un joint torique, assemblé sur ledit insert creux.

Selon un premier mode de réalisation avantageux, ledit réservoir ne contient qu'une seule dose de produit fluide, distribuée en un seul actionnement.

Selon un second mode de réalisation avantageux, ledit réservoir contient plusieurs doses de produit fluide distribuées en plusieurs actionnements successifs.

Avantageusement, ledit réservoir contient deux doses de produit fluide distribuées en deux actionnements successifs.

Avantageusement, ladite chambre de dosage comporte une troisième ouverture prévue à l'extrémité axiale inférieure dudit corps de pompe, ladite troisième ouverture comportant un organe d'obturation, tel qu'une bille, formant un clapet d'entrée pour ladite chambre de dosage permettant le remplissage de celle-ci après chaque actionnement.

La présente invention a également pour objet un procédé d'amorçage d'un dispositif de distribution de produit fluide, comportant les étapes suivantes:
- fournir un réservoir contenant au moins une dose de produit fluide,
- fournir un distributeur comportant un corps de pompe contenant une chambre de dosage, et une tête de distribution comportant un orifice de distribution, ladite tête de distribution étant déplaçable axialement par rapport audit corps de pompe entre une position de repos et une position d'actionnement, un piston, solidaire de ladite tête de distribution, étant adapté à coulisser dans ladite chambre de dosage entre une position de repos et une position d'actionnement, ladite chambre de dosage comportant au moins deux ouvertures, une première ouverture permettant le remplissage de ladite chambre de dosage et une seconde ouverture reliant ladite chambre de dosage audit orifice de distribution, ladite première ouverture étant réalisée dans une partie cylindrique dudit corps de pompe dans laquelle ledit piston coulisse lors de l'actionnement, ladite première ouverture reliant ladite chambre de dosage audit réservoir lorsque ledit piston est en position de repos, et ladite première ouverture isolant ladite chambre de dosage dudit réservoir lorsque ledit piston se déplace hors de sa position de repos,
ledit procédé comportant l'étape d'assembler ledit distributeur sur ledit réservoir, en insérant au moins partiellement ledit corps de pompe dans ledit réservoir avec ledit piston en position de repos, cette insertion déplaçant le produit fluide contenu dans ledit réservoir, ledit produit fluide déplacé pénétrant dans ladite chambre de dosage à travers ladite première ouverture, pour ainsi amorcer le dispositif.

Ces caractéristiques et avantages et d'autres de la présente invention apparaitront plus clairement au cours de la description détaillée suivante, faite en référence aux dessins joints, donnés à titre d'exemples non limitatifs, et sur lesquels :
La figure 1 est une vue schématique en section transversale d'un dispositif de distribution de produit fluide selon un mode de réalisation avantageux, lors de l'assemblage du distributeur sur le réservoir,
La figure 2 est une vue similaire à celle de la figure 1, lors du premier actionnement,
La figure 3 est une vue similaire à celle de la figure 2, après le premier actionnement,
La figure 4 est une vue similaire à celle de la figure 3, après chargement de la chambre de dosage avec une nouvelle dose de produit fluide,
La figure 5 est une vue schématique agrandie d'une partie de la figure 1,
La figure 6 est une vue schématique découpée d'un réservoir avant assemblage du distributeur, et
La figure 7 est une vue schématique en perspective d'un ressort plastique pouvant remplacer le ressort métallique des figures 1 à 4.

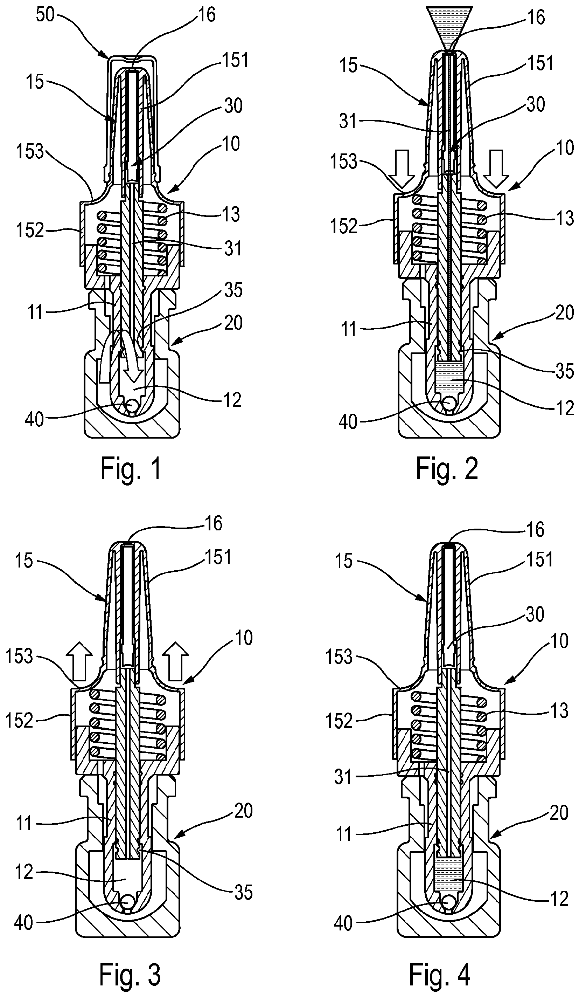

Les termes "axial" et "radial" se réfèrent à l'axe central longitudinal du dispositif. Les termes "amont" et "aval" se réfèrent à la direction d'écoulement du fluide lors de l'actionnement. Les termes "haut" et "bas" se réfèrent à la position droite du dispositif représentée sur les figures 1 à 4.

Dans le mode de réalisation avantageux des figures 1 à 5, un distributeur 10 est assemblé sur un réservoir 20 contenant du produit fluide à distribuer, typiquement un liquide de type pharmaceutique.

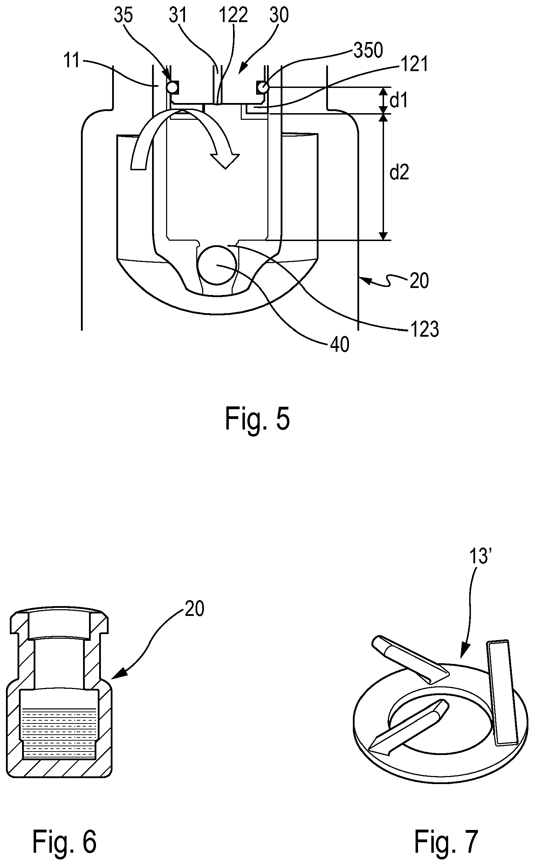

Le réservoir 20 est avantageusement formé par un corps creux et borgne, comportant une seule ouverture et contenant une ou plusieurs dose(s) de produit fluide à distribuer. Un exemple de réalisation de ce réservoir 20 adapté au mode de réalisation des figures 1 à 5 est représenté de manière isolée sur la figure 6.

La présente invention s'applique à un dispositif unidose ne contenant qu'une seule dose de produit fluide dans le réservoir 20, mais elle est particulièrement adaptée pour des dispositifs dits bidoses, où le réservoir 20 contient deux doses de produit fluide, distribuées en deux actionnements successifs. Eventuellement, le réservoir 20 pourrait contenir plus de deux doses, par exemple trois ou quatre doses.

Le distributeur 10 comprend un corps de pompe 11 contenant une chambre de dosage 12, et une tête de distribution 15 comportant un orifice de distribution 16, de préférence orienté axialement. Cet orifice de distribution 16 sert à distribuer une dose de produit fluide hors de ladite tête de distribution 15 lors de l'actionnement du dispositif par un utilisateur. La tête de distribution 15 est déplaçable axialement par rapport au corps de pompe 11 entre une position de repos et une position d'actionnement.

Au repos, un capuchon de protection amovible 50 peut être disposé sur la tête de distribution 15 pour protéger l'orifice de distribution 16, comme visible sur la figure 1.

Un ressort 13 est disposé entre le corps de pompe 11 et la tête de distribution 15 pour solliciter cette dernière vers sa position de repos.

Dans le mode de réalisation des figures 1 à 4, le ressort 13 est du type classique à boudin, généralement métallique.

En variante, on peut envisager de remplacer le ressort 13 par un élément élastiquement déformable 13' en plastique. La figure 7 montre un exemple d'un tel élément élastique 13'.

La tête de distribution 15 comporte un embout nasal allongé 151 comportant à son extrémité axiale ledit orifice de distribution 16, ainsi qu'un corps latéral 152, relié audit embout nasal 151 par une bride 153 environ radiale.

Un insert creux 30 est disposé dans la tête de distribution 15, ledit insert creux 30 définissant un canal d'expulsion 31.

Avantageusement, un profil de pulvérisation peut être prévu directement en amont dudit orifice de distribution 16, entre ledit insert 30 et la paroi de fond de la tête de distribution 15.

Du côté opposé, l'insert creux 30 comporte un piston 35 adapté à coulisser dans la chambre de dosage 12 du corps de pompe 11 entre une position de repos et une position d'actionnement. Dans l'exemple des figures, un joint 350, notamment un joint torique, est assemblé sur l'insert creux 30 pour former le piston, comme plus particulièrement visible sur la figure 5. En variante, le piston 35 pourrait être formé monobloc avec l'insert creux 30.

L'assemblage du distributeur 10 sur le réservoir 20 peut être réalisé d'une quelconque manière connue, par exemple en utilisant une bague de fixation pour fixer le corps de pompe 11 sur un col du réservoir.

Selon l'invention, la chambre de dosage 12 comporte au moins deux ouvertures 121, 122, 123. Ces ouvertures sont distinctes, et ont chacune une fonction spécifique.

Dans le cas d'un unidose, lorsque le réservoir 20 ne contient qu'une seule dose de produit fluide, il y a deux ouvertures 121 et 122.

Dans le cas d'un bidose ou multidose, lorsque le réservoir 20 contient au moins deux dose de produit fluide, il y a trois ouvertures 121, 122 et 123.

La première ouverture 121 est réalisée dans la partie cylindrique du corps de pompe 11 dans laquelle le piston 35 coulisse lors de l'actionnement.

Cette première ouverture 121 relie la chambre de dosage 12 au réservoir 20 lorsque le piston 35 est en position de repos. Lors de l'actionnement, le piston 35 coulisse axialement vers le bas dans le corps de pompe 11. Eventuellement, notamment dans le cas d'un bidose, après distribution de la première dose, le piston 35 remonte axialement pour revenir dans sa position de repos. A la fin de cette course de retour, la première ouverture 121 est ouverte à nouveau.

Comme visible sur la figure 5, la première ouverture 121 est de préférence disposée au repos à proximité du piston 35, de sorte qu'après une petite course d'actionnement initiale d1, illustrée sur la figure 5, le piston 35 dépasse la première ouverture 121, et pour la suite de la course d'actionnement d2, cette première ouverture 121 est donc fermée. En d'autres termes, un bord inférieur de la première ouverture 121 est disposé au repos à une distance d1 du piston 35, de sorte que, lors de l'actionnement, ladite première ouverture 121 est fermée après une course initiale correspondant à ladite distance d1. Avantageusement, la distance d1 forme moins de 30% de la course d'actionnement totale d1 + d2 du piston 35, de préférence environ 20%.

Avantageusement, la première ouverture 121 comporte une ou plusieurs fentes formées dans la partie cylindrique du corps de pompe 11. Le nombre de fentes, qui pourraient aussi être des trous, peut être quelconque, par exemple un, deux, trois ou quatre fentes.

La seconde ouverture 122 est axiale et est prévue au niveau de l'extrémité axiale inférieure de l'insert creux 30. La seconde ouverture 122 relie ainsi la chambre de dosage 12 au canal d'expulsion 31.

La troisième ouverture 123 est axiale et est prévue à l'extrémité axiale inférieure du corps de pompe 11. Cette troisième ouverture n'est prévue que si le dispositif est un bidose ou un multidose. Un organe d'obturation 40, tel qu'une bille, est prévu pour fermer la troisième ouverture 123 dès le début de l'actionnement, et pour ouvrir la troisième ouverture 123 dès le début de la course de retour du piston 35, quand celui-ci revient de sa position d'actionnement vers sa position de repos. Cette troisième ouverture 123 ensemble avec l'organe d'obturation 40 forme donc un clapet d'entrée classique pour la chambre de dosage 12, permettant le remplissage de celle-ci après chaque actionnement de la pompe du fait de la dépression générée par le piston 35 qui remonte vers sa position de repos.

Dans l'exemple représenté sur les figures, la chambre de dosage 12 ne comporte pas de clapet de sortie au niveau de la seconde ouverture 122, et l'orifice de distribution 16 ne comporte pas d'obturateur. La chambre de dosage 12 est donc reliée en permanence à l'atmosphère. Néanmoins, le rapport des aires des sections transversale du canal d'expulsion 31 et de la troisième ouverture 123 est tel, que la dépression est générée quand même dans la chambre de dosage 12 lorsque le piston 35 retourne vers sa position de repos. Ainsi par exemple, le diamètre du canal d'expulsion 31 peut être de 0,3 mm, ce qui donne une aire de la section transversale de 0,7 mm², alors que le diamètre de la troisième ouverture 123 est typiquement d'environ 3 mm, ce qui donne une aire de la section transversale de 7 mm², soit un rapport de 1 à 100. Néanmoins, en variante, on pourrait envisager un clapet de sortie au niveau de la seconde ouverture 122 de la chambre de dosage, ou un obturateur au niveau de l'orifice de distribution 16.

Ainsi, l'invention permet de réaliser un dispositif sans amorçage.

Lors de l'assemblage du distributeur 10 sur le réservoir 20, la tête de distribution 15 et le piston 35 sont en position de repos par rapport au corps de pompe 11. La première ouverture 121 est donc ouverte. Ainsi, lorsque le distributeur 10 est assemblé sur le réservoir 20, le corps de pompe 11 pénètre dans le réservoir 20 et de ce fait déplace le produit fluide contenu dans le réservoir 20. Le volume de produit fluide déplacé correspond au volume qu'occupe le corps de pompe 11 dans le réservoir après assemblage. Le produit fluide ainsi déplacé pénètre donc dans la chambre de dosage 12 via la première ouverture 121, comme illustré par la flèche sur les figures 1 et 5. L'assemblage se faisant en position droite, la chambre de dosage 12 se remplit jusqu'au bord de la première ouverture 121, définissant ainsi la dose à distribuer. La pompe est donc amorcée dès l'assemblage, et il n'y a pas besoin de réaliser des actionnements d'amorçage avant l'utilisation du dispositif.

Lors du premier actionnement, l'utilisateur place deux doigts sur la bride radiale 153 formée sur la tête de distribution 15, et appuie avec le pouce sur le fond du réservoir 20. Lors d'un tel actionnement, le réservoir 20 est donc poussé axialement en direction de l'orifice de distribution 16, de sorte que le piston 35 coulisse dans la chambre de dosage 12. La première ouverture 121 est fermée après la petite course initiale d1 et la troisième ouverture 123 est fermée par la pression exercée par le produit fluide sur l'organe d'obturation 40 formant clapet. Le contenu de la chambre de dosage 12 est ainsi distribué à travers la seconde ouverture 122 en direction de l'orifice de distribution 16.

Si le dispositif est un bidose ou un multidose, lorsque l'utilisateur relâche la pression sur la tête de distribution 15 après le premier actionnement, le ressort 13 ramène le piston 35 vers sa position de repos. Il se crée ainsi une dépression dans la chambre de dosage 12, qui ouvre la troisième ouverture 123 pour permettre le remplissage de la chambre de dosage 12 à partir du réservoir 20 à travers cette troisième ouverture 123.

Le dispositif est alors prêt pour un second actionnement.

Ainsi, la première ouverture 121 assure le transfert de la première dose dans la chambre de dosage 12 lors de l'assemblage, et la troisième ouverture 123 assure le transfert de la seconde dose (et éventuellement d'autres doses additionnelles) dans la chambre de dosage 12 après distribution de la première dose.

Bien entendu, des variantes de réalisation sont envisageables, sans sortir du cadre de la présente invention, tel que défini par les revendications annexées.

## Revendications

1. Dispositif de produit fluide comprenant un distributeur (10) assemblé sur un réservoir (20) contenant du produit fluide, ledit distributeur (10) comportant un corps de pompe (11) contenant une chambre de dosage (12), et une tête de distribution (15) comportant un orifice de distribution (16), ladite tête de distribution (15) étant déplaçable axialement par rapport audit corps de pompe (11) entre une position de repos et une position d'actionnement, un piston (35), solidaire de ladite tête de distribution (15), étant adapté à coulisser dans ladite chambre de dosage (12) entre une position de repos et une position d'actionnement, ladite chambre de dosage (12) comportant au moins deux ouvertures (121, 122, 123), une première ouverture (121) permettant le remplissage de ladite chambre de dosage (12) et une seconde ouverture (122) reliant ladite chambre de dosage (12) audit orifice de distribution (16), **caractérisé en ce que** ladite première ouverture (121) est réalisée dans une partie cylindrique dudit corps de pompe (11) dans laquelle ledit piston (35) coulisse lors de l'actionnement, ladite première ouverture (121) reliant ladite chambre de dosage (12) audit réservoir (20) lorsque ledit piston (35) est en position de repos, et ladite première ouverture (121) isolant ladite chambre de dosage (12) dudit réservoir (20) lorsque ledit piston (35) se déplace hors de sa position de repos, ladite chambre de dosage (12) étant remplie à travers ladite première ouverture (121) lorsque ledit distributeur (10) est assemblé sur ledit réservoir (20).

2. Dispositif selon la revendication 1, dans lequel un bord inférieur de ladite première ouverture (121) est disposé au repos à une distance (d1) dudit piston (35), de sorte que, lors de l'actionnement, ladite première ouverture (121) est fermée après une course initiale correspondant à ladite distance (d1).

3. Dispositif selon la revendication 2, dans lequel ladite distance (d1) forme moins de 30% de la course d'actionnement totale dudit piston (35), de préférence environ 20%.

4. Dispositif selon l'une quelconque des revendications précédentes, dans lequel ladite première ouverture (121) comporte une ou plusieurs fentes formée(s) dans la partie cylindrique dudit corps de pompe (11).

5. Dispositif selon l'une quelconque des revendications précédentes, dans lequel un insert creux (30) est disposé dans ladite tête de distribution (15), ledit insert creux (30) définissant un canal d'expulsion (31) reliant lors de l'actionnement ladite chambre de dosage (12) audit orifice de distribution (16), ledit insert creux (30) comportant ledit piston (35).

6. Dispositif selon la revendication 5, dans lequel ledit piston (35) est formé par un joint (350), notamment un joint torique, assemblé sur ledit insert creux (30).

7. Dispositif selon l'une quelconque des revendications précédentes, dans lequel ledit réservoir ne contient qu'une seule dose de produit fluide, distribuée en un seul actionnement.

8. Dispositif selon l'une quelconque des revendications 1 à 6, dans lequel ledit réservoir contient plusieurs doses de produit fluide distribuées en plusieurs actionnements successifs.

9. Dispositif selon la revendication 8, dans lequel ledit réservoir contient deux doses de produit fluide distribuées en deux actionnements successifs.

10. Dispositif selon la revendication 8 ou 9, dans lequel ladite chambre de dosage (12) comporte une troisième ouverture (123) prévue à l'extrémité axiale inférieure dudit corps de pompe (11), ladite troisième ouverture (123) comportant un organe d'obturation (40), tel qu'une bille, formant un clapet d'entrée pour ladite chambre de dosage (12) permettant le remplissage de celle-ci après chaque actionnement.

11. Procédé d'amorçage d'un dispositif de distribution de produit fluide, comportant les étapes suivantes:
- fournir un réservoir (20) contenant au moins une dose de produit fluide,
- fournir un distributeur (10) comportant un corps de pompe (11) contenant une chambre de dosage (12), et une tête de distribution (15) comportant un orifice de distribution (16), ladite tête de distribution (15) étant déplaçable axialement par rapport audit corps de pompe (11) entre une position de repos et une position d'actionnement, un piston (35), solidaire de ladite tête de distribution (15), étant adapté à coulisser dans ladite chambre de dosage (12) entre une position de repos et une position d'actionnement, ladite chambre de dosage (12) comportant au moins deux ouvertures (121, 122, 123), une première ouverture (121) permettant le remplissage de ladite chambre de dosage (12) et une seconde ouverture (122) reliant ladite chambre de dosage (12) audit orifice de distribution (16), ladite première ouverture (121) étant réalisée dans une partie cylindrique dudit corps de pompe (11) dans laquelle ledit piston (35) coulisse lors de l'actionnement, ladite première ouverture (121) reliant ladite chambre de dosage (12) audit réservoir (20) lorsque ledit piston (35) est en position de repos, et ladite première ouverture (121) isolant ladite chambre de dosage (12) dudit réservoir (20) lorsque ledit piston (35) se déplace vers sa position d'actionnement,
**caractérisé en ce que** ledit procédé comporte l'étape d'assembler ledit distributeur (10) sur ledit réservoir (20), en insérant ledit corps de pompe (11) dans ledit réservoir (20) avec ledit piston (35) en position de repos, cette insertion déplaçant le produit fluide contenu dans ledit réservoir (20), ledit produit fluide déplacé pénétrant dans ladite chambre de dosage (12) à travers ladite première ouverture (121), pour ainsi amorcer le dispositif.

## Patentansprüche

1. Vorrichtung für fluides Produkt, umfassend einen Spender (10), der an einem Behälter (20) angebracht ist, der fluides Produkt enthält, wobei der Spender (10) einen Pumpenkörper (11) aufweist, der eine Dosierkammer (12) enthält, und einen Spenderkopf (15), der eine Abgabeöffnung (16) aufweist, wobei der Spenderkopf (15) axial relativ zum Pumpenkörper (11) zwischen einer Ruhestellung und einer Betätigungsstellung verschiebbar ist, wobei ein mit dem Spenderkopf (15) fest verbundener Kolben (35) in der Dosierkammer (12) zwischen einer Ruhestellung und einer Betätigungsstellung verschiebbar ist, wobei die Dosierkammer (12) mindestens zwei Öffnungen (121, 122, 123) aufweist, wobei eine erste Öffnung (121) das Befüllen der Dosierkammer (12) ermöglicht und eine zweite Öffnung (122) die Dosierkammer (12) mit der Abgabeöffnung (16) verbindet,
**dadurch gekennzeichnet, dass** die erste Öffnung (121) in einem zylindrischen Teil des Pumpenkörpers (11) ausgebildet ist, in dem der Kolben (35) bei der Betätigung gleitet, wobei die erste Öffnung (121) die Dosierkammer (12) mit dem Behälter (20) verbindet, wenn sich der Kolben (35) in Ruhestellung befindet, und wobei die erste Öffnung (121) die Dosierkammer (12) vom Behälter (20) isoliert, wenn sich der Kolben (35) aus seiner Ruhestellung herausbewegt, wobei die Dosierkammer (12) durch die erste Öffnung (121) befüllt wird, wenn der Spender (10) auf dem Behälter (20) angebracht ist.

2. Vorrichtung nach Anspruch 1, wobei eine Unterkante der ersten Öffnung (121) in Ruhe in einem Abstand (d1) vom Kolben (35) angeordnet ist, so dass bei Betätigung die erste Öffnung (121) nach einem Anfangshub, der dem Abstand (d1) entspricht, geschlossen wird.

3. Vorrichtung nach Anspruch 2, wobei der Abstand (d1) weniger als 30 % des gesamten Betätigungshubs des Kolbens (35) bildet, vorzugsweise etwa 20 %.

4. Vorrichtung nach einem der vorstehenden Ansprüche, wobei die erste Öffnung (121) einen oder mehrere Schlitze aufweist, die in dem zylindrischen Teil des Pumpenkörpers (11) ausgebildet sind.

5. Vorrichtung nach einem der vorstehenden Ansprüche, wobei ein hohler Einsatz (30) in dem Spenderkopf (15) angeordnet ist, wobei der hohle Einsatz (30) einen Ausstoßkanal (31) definiert, der bei Betätigung die Dosierkammer (12) mit der Abgabeöffnung (16) verbindet, wobei der hohle Einsatz (30) den Kolben (35) aufweist.

6. Vorrichtung nach Anspruch 5, wobei der Kolben (35) von einer Dichtung (350), insbesondere einem O-Ring, gebildet wird, die auf dem hohlen Einsatz (30) angebracht ist.

7. Vorrichtung nach einem der vorstehenden Ansprüche, wobei der Behälter nur eine einzige Dosis des fluiden Produkts enthält, die bei einer einzigen Betätigung abgegeben wird.

8. Vorrichtung nach einem der Ansprüche 1 bis 6, wobei der Behälter mehrere Dosen des fluiden Produkts enthält, die bei mehreren aufeinanderfolgenden Betätigungen abgegeben werden.

9. Vorrichtung nach Anspruch 8, wobei der Behälter zwei Dosen des fluiden Produkts enthält, die bei zwei aufeinanderfolgenden Betätigungen abgegeben werden.

10. Vorrichtung nach Anspruch 8 oder 9, wobei die Dosierkammer (12) eine dritte Öffnung (123) aufweist, die am unteren axialen Ende des Pumpenkörpers (11) vorgesehen ist, wobei die dritte Öffnung (123) ein Verschlusselement (40) wie eine Kugel aufweist, das ein Einlassventil für die Dosierkammer (12) bildet, das die Befüllung derselben nach jeder Betätigung gestattet.

11. Verfahren zum Aktivieren einer Vorrichtung zur Abgabe eines fluiden Produkts, das die folgenden Schritte aufweist:
- Bereitstellen eines Behälters (20), der mindestens eine Dosis eines fluiden Produkts enthält,
- Bereitstellen eines Spenders (10), der einen Pumpenkörper (11) aufweist, der eine Dosierkammer (12) enthält, und einen Spenderkopf (15), der eine Abgabeöffnung (16) aufweist, wobei der Spenderkopf (15) axial relativ zum Pumpenkörper (11) zwischen einer Ruhestellung und einer Betätigungsstellung verschiebbar ist, wobei ein mit dem Spenderkopf (15) fest verbundener Kolben (35) in der Dosierkammer (12) zwischen einer Ruhestellung und einer Betätigungsstellung verschiebbar ist, wobei die Dosierkammer (12) mindestens zwei Öffnungen (121, 122, 123) aufweist, wobei eine erste Öffnung (121) das Befüllen der Dosierkammer (12) ermöglicht und eine zweite Öffnung (122) die Dosierkammer (12) mit der Abgabeöffnung (16) verbindet, wobei die erste Öffnung (121) in einem zylindrischen Teil des Pumpenkörpers (11) ausgebildet ist, in dem der Kolben (35) bei der Betätigung gleitet, wobei die erste Öffnung (121) die Dosierkammer (12) mit dem Behälter (20) verbindet, wenn sich der Kolben (35) in Ruhestellung befindet, und die erste Öffnung (121) die Dosierkammer (12) vom Behälter (20) isoliert, wenn sich der Kolben (35) in seine Betätigungsstellung verlagert,
**dadurch gekennzeichnet, dass** das Verfahren den Schritt des Anbringens des Spenders (10) auf dem Behälter (20) umfasst, indem der Pumpenkörper (11) mit dem Kolben (35) in Ruhestellung in den Behälter (20) eingesetzt wird, wobei sich bei diesem Einsetzen das in dem Behälter (20) enthaltene fluide Produkt verlagert, wobei das verlagerte fluide Produkt durch die erste Öffnung (121) in die Dosierkammer (12) eindringt, um so die Vorrichtung zu aktivieren.

## Claims

1. Fluid product device comprising a dispenser (10) joined to a reservoir (20) which contains fluid product, said dispenser (10) comprising a pump body (11) which contains a dosing chamber (12), and a dispensing head (15) comprising a dispensing aperture (16), said dispensing head (15) being axially movable with respect to said pump body (11) between a rest position and an actuation position, a plunger (35), which is secured to said dispensing head (15), being designed to slide in said dosing chamber (12) between a rest position and an actuation position, said dosing chamber (12) comprising at least two openings (121, 122, 123), a first opening (121) for filling said dosing chamber (12) and a second opening (122) connecting said dosing chamber (12) to said dispensing aperture (16), **characterised in that** said first opening (121) is made in a cylindrical portion of said pump body (11) in which said plunger (35) slides during actuation, said first opening (121) connecting said dosing chamber (12) to said reservoir (20) when said plunger (35) is in the rest position, and said first opening (121) isolating said dosing chamber (12) from said reservoir (20) when said plunger (35) moves out of its rest position, said dosing chamber (12) being filled through said first opening (121) when said dispenser (10) is joined to said reservoir (20).

2. Device according to claim 1, wherein a lower edge of said first opening (121) is disposed at rest at a distance (d1) from said plunger (35), such that during actuation, said first opening (121) is closed after an initial stroke corresponding to said distance (d1).

3. Device according to claim 2, wherein said distance (d1) forms less than 30% of the total actuation stroke of said plunger (35), preferably around 20%.

4. Device according to any one of preceding claims, wherein said first opening (121) comprises one or more slots formed in the cylindrical portion of said pump body (11).

5. Device according to any one of preceding claims, wherein a hollow insert (30) is disposed in said dispensing head (15), said hollow insert (30) defining an expulsion channel (31) connecting, during actuation, said dosing chamber (12) to said dispensing aperture (16), said hollow insert (30) comprising said plunger (35).

6. Device according to claim 5, wherein said piston (35) is formed by a seal (350), in particular an O-ring, joined to said hollow insert (30).

7. Device according to any one of the preceding claims, wherein said reservoir only contains a single dose of fluid product, dispensed in a single actuation.

8. Device according to any one of claims 1 to 6, wherein said reservoir contains several doses of fluid product dispensed in several successive actuations.

9. Device according to claim 8, wherein said reservoir contains two doses of fluid product dispensed in two successive actuations.

10. Device according to claim 8 or 9, wherein said dosing chamber (12) comprises a third opening (123) provided at the lower axial end of said pump body (11), said third opening (123) comprising a stopper member (40), such as a ball, forming an inlet valve for said dosing chamber (12) for filling it after each actuation.

11. Method for priming a device for dispensing a fluid product, comprising the following steps:
- providing a reservoir (20) which contains at least one dose of fluid product;
- providing a dispenser (10) comprising a pump body (11) which contains a dosing chamber (12), and a dispensing head (15) comprising a dispensing aperture (16), said dispensing head (15) being axially movable with respect to said pump body (11) between a rest position and an actuation position, a plunger (35), which is secured to said dispensing head (15), being designed to slide in said dosing chamber (12) between a rest position and an actuation position, said dosing chamber (12) comprising at least two openings (121, 122, 123), a first opening (121) for filling said dosing chamber (12) and a second opening (122) connecting said dosing chamber (12) to said dispensing aperture (16), said first opening (121) being made in a cylindrical portion of said pump body (11) in which said plunger (35) slides during actuation, said first opening (121) connecting said dosing chamber (12) to said reservoir (20) when said piston (35) is in the rest position, and said first opening (121) isolating said dosing chamber (12) from said reservoir (20) when said plunger (35) is moved to its actuation position,
**characterised in that** said method comprises the step of joining said dispenser (10) to said reservoir (20), by inserting said pump body (11) in said reservoir (20) with said plunger (35) in the rest position, this insertion moving the fluid product contained in said reservoir (20), said moved fluid product penetrating inside said dosing chamber (12) through said first opening (121), to thus prime the device.
